(19) Europäisches Patentamt
European Patent Office
Office européen des brevets

(11) Publication number: 0 097 323 A2

(12) EUROPEAN PATENT APPLICATION

(21) Application number: 83105857.3

(22) Date of filing: 15.06.83

(51) Int. Cl.³: C 07 D 417/04
C 07 D 417/14, A 61 K 31/44

(30) Priority: 22.06.82 US 391061

(43) Date of publication of application: 04.01.84 Bulletin 84/1

(84) Designated Contracting States: AT BE CH DE FR GB IT LI LU NL SE

(71) Applicant: SCHERING CORPORATION
2000 Galloping Hill Road
Kenilworth, New Jersey 07033(US)

(72) Inventor: Witkowski, Joseph Theodore
5 Martha Drive
Morristownship New Jersey 07960(US)

(72) Inventor: Sunday, Brook Robert
124 Manito Avenue
Oakland New Jersey 07436(US)

(74) Representative: Antony, Fritz, Dr. et al,
P.O. Box 601 Winkelriedstrasse 35
CH-6002 Lucerne(CH)

(54) 2-(4-Pyridyl)-thiazole derivatives.

(57) This invention relates to 2-(4-pyridyl)-thiazole derivatives of the general formula

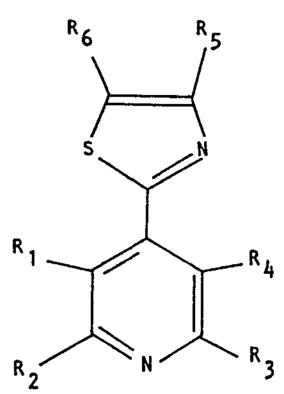

(wherein the substituents $R_1$ to $R_6$ are as defined in the specification). The invention also relates to processes for preparing such compounds and to pharmaceutical compositions containing them. The compounds are useful in increasing cardiac contractility and some of the compounds exhibit bronchodilator activity.

EP 0 097 323 A2

# 2-(4-PYRIDYL)-THIAZOLE DERIVATIVES

This invention relates to 2-(4-pyridyl)-thiazole derivatives of the general formula I

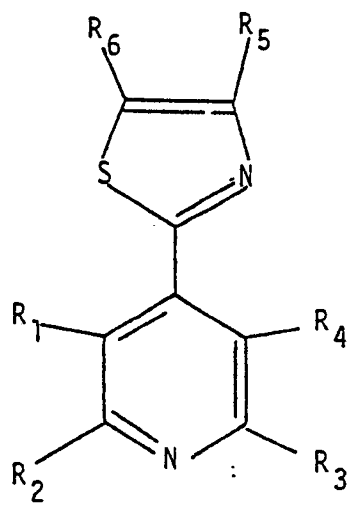

(I),

wherein $R_1$, $R_2$, $R_3$ and $R_4$ are independently selected from hydrogen, halogen, loweralkyl, loweralkoxy, hydroxy and amino;

$R_5$ and $R_6$ are independently selected from hydrogen, loweralkoxy, hydroxyloweralkyl, loweralkyl, hydroxy, $-CF_3$, $-CN$, $-NR_7R_8$, 2-imidazolyl, 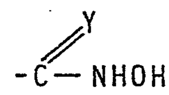 and 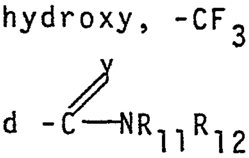 , with the provisos that

(i) $R_5$ and $R_6$ are not hydrogen simultaneously;

(ii) when $R_1$,$R_2$,$R_3$,$R_4$ and $R_6$ are all hydrogen, $R_5$ is not loweralkyl; and

(iii) when $R_1$,$R_2$,$R_3$ and $R_4$ are all hydrogen and $R_5$ is hydroxy, $R_6$ is not loweralkyl;

whereby in the above definitions $R_7$ and $R_8$ are independently selected from hydrogen and loweralkyl;

$R_{11}$ and $R_{12}$ are independently selected from hydrogen, loweralkyl and hydroxyloweralkyl; and

Y represents O, S or NH;

the pyridyl-N-oxides therof and pharmaceutically acceptable salts of such compounds.

The compounds are useful in increasing cardiac contractility and, in addition, certain compounds exhibit bronchodilator activity.

The preferred definitions for $R_1$,$R_2$,$R_3$ and $R_4$ are hydrogen and loweralkyl, preferably methyl. Most preferred is hydrogen.

Preferably one of $R_5$ and $R_6$ is hydrogen and the other is selected from amino, hydroxy, cyano, 2-imidazolyl, $-\overset{Y}{\overset{\|}{C}}-NHOH$ and $-\overset{Y}{\overset{\|}{C}}-NR_{11}R_{12}$ wherein Y is O, NH or S and $R_{11}$ and $R_{12}$ are independantly hydrogen, hydroxyloweralkyl or loweralkyl. Most preferably $R_5$ is $-\overset{O}{\overset{\|}{C}}-NH_2$ or $-\overset{S}{\overset{\|}{C}}-NH_2$.

Compounds of the general formula II

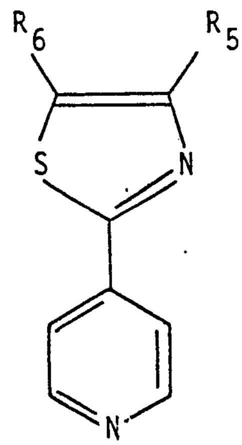

(II),

wherein one of $R_5$ and $R_6$ is hydrogen and the other is selected from CN, 2-imidazolyl, $-\overset{O}{\overset{\|}{C}}NHR'$ or $-\overset{Y'}{\overset{\|}{C}}NHR''$, wherein R' is H, lower alkyl having from 2 to 6 carbon atoms or hydroxyloweralkyl having from 1 to 6 carbon atoms, Y' is S or NH and R" is hydrogen, hydroxy, loweralkyl having from 1 to 6 carbon atoms or hydroxyloweralkyl having from 1 to 6 carbon atoms, are particularly useful as bronchodilators.

The term "loweralkyl" includes branched- and straight-chain alkyl groups having 1 to 6 carbon atoms, such as methyl, ethyl, n-propyl, iso-propyl, t-butyl and n-hexyl. Equally the term "loweralkoxy" includes both branched- and straight-chain groups having 1 to 6 carbon atoms, such as, for example, methoxy, ethoxy, isopropoxy, t-butoxy and n-hexoxy.

The term "halogen" refers to F, Cl, Br and I.

The hydroxyloweralkyl groups are also such having 1 to 6 carbon atoms, e.g. 2-hydroxyethyl, 2-hydroxy-n-propyl, 3-hydroxy-n-propyl, etc.

The term "pharmaceutically acceptable salts" includes the pharmaceutically acceptable acid addition salts derived from various organic and inorganic acids, such as sulfuric, phosphoric, hydrochloric, hydrobromic, sulfamic, citric, lactic, oleic, succinic, tartaric, cinnamic, fumaric, acetic, benzoic, gluconic and ascorbic acid.

Some of the compounds of this invention may exist in tautomeric forms. Thus, for example, compounds of formula I having a hydroxy group adjacent to the nitrogen atom of the pyridine ring ($R_2$ or $R_3$ being OH) may exist in two forms:

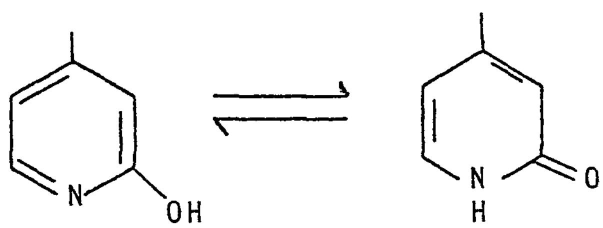

The same applies to compounds of formula I wherein one of $R_5$ and $R_6$ is hydroxy, e.g.:

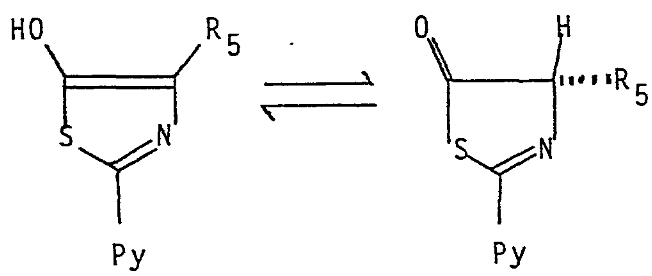

Also compounds wherein $R_5$ and/or $R_6$ are $-C(=NH)-NHOH$ may exist in the tautomeric form $-C(=NOH)-NH_2$.

The definition of compounds of formula I is meant to include all such tautomeric forms. (Py represents an appropriately $R_1$,$R_2$,$R_3$ and/or $R_4$ substituted 4-pyridine ring).

Various pyridyl-thiazoles are known in the art. For example, United States Patents 3,821,384 and 3,842,172 describe various 2-(3-pyridyl)-thiazoles useful as anti-aggression agents. United States Patent 3,852,293 describes certain 2-(3-pyridyl)-thiazoles as antiinflammatory agents. Liebigs Ann. Chem. 717, 148-153 (1968) and Chem. Abstract 70:37693Z teaches the structure of various 2-(4-pyridyl)-thiazoles which, however, do not fall under the definition given for formula I above.

United Kingdom Patent 1,382,854 describes various 2-(3-pyridyl)-thiazole derivatives as possessing insecticidal activity.

Journal of Organic Chemistry, 22, 984-986 (1957) describes the 4-methyl-2-(4-pyridyl)-thiazole and 4-hydroxy-5-methyl-2-(4-pyridyl)-thiazole.

Journal of Medicinal Chemistry, 23, 65-70 (1980) also describes the 4-hydroxy-5-methyl-2-(4-pyridyl)-thiazole.

The compounds of this invention may be prepared according to processes known for the preparation of compounds having a similar structure, e.g. as described in the literature cited hereinabove or hereinafter.

A: One basic approach is characterized in that a compound of formula III

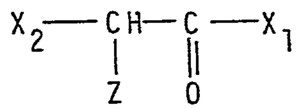

(III),

wherein Z is halogen and either one of $X_1$ and $X_2$ is the group $-COOR_{14}$ wherein $R_{14}$ is hydrogen or loweralkyl and the other is as defined for $R_5$ respectively $R_6$, or both $X_1$ and $X_2$ represent the group $-COOR_{14}$ is reacted with a thioisonicotinamide of the formula IV

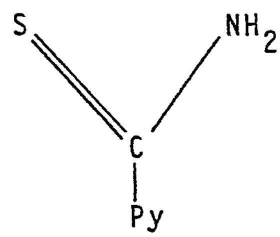

(IV),

wherein Py represents $R_1$,$R_2$,$R_3$ and/or $R_4$ substituted 4-pyridyl, resulting in a compound of formula V

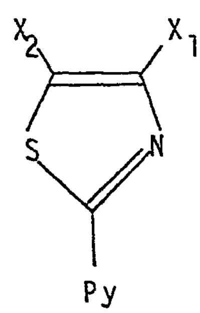

(V)

wherein $X_1$,$X_2$ and Py are as above defined,

that any $-COOR_{14}$ group present in a compound of formula V is transformed into the group $-C-NR_{11}R_{12}$ or the group 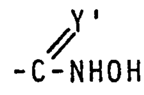, wherein Y' is O or S, or the group $-NR_7R_8$, whereby $R_7$,$R_8$,$R_{11}$ and $R_{12}$ are as above defined,

and that a so-obtained compound of formula I, if desired, is subjected to one or more of the following subsequent reactions at the substituents $R_5$ and/or $R_6$:

(i) alkylation of hydroxy to alkoxy;

(ii) transformation of 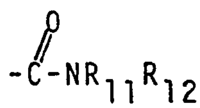 into 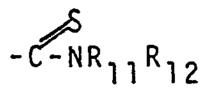;

(iii) transformation of 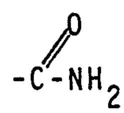 into CN;

(iv) transformation of CN into 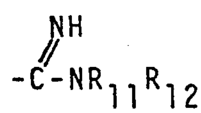;

(v) transformation of CN into 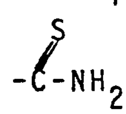

(vi) transformation of CN into 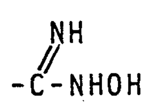 respectively 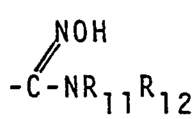 ;

(vii) transformation of CN into 2-imidazolyl;

(viii) transformation of any compound of formula I into its 4-pyridyl-N-oxide;

(ix) transformation of a compound of formula I into its acid addition salt.

Reaction A above is a conventional condensation reaction e.g. as described in Chem. Abstracts 70:37693Z and Liebigs Ann. Chem. 717, 148-153 (1968). The reaction can be conducted by adding an essentially equimolar amount of the appropriate thioisonicotinamide to the reagent of formula III. The reaction may be conducted in the liquid phase employing inert protic or aprotic solvents such as toluene, hydroxylic solvents (e.g. methanol, ethanol, water and the like), or a mixture of water and another hydroxylic solvent. Reaction pressure is not critical and for convenience the reaction is generally conducted at atmospheric pressure. The reaction is generally conducted at from 20° to 100°C., although preferably at the boiling point of the solvent employed, and is generally complete from within 1 to 72 hours. The resulting 2-(pyridyl)-thiazole V or its acid addition salt, e.g. HCl-salt, is then isolated and purified by conventional procedures such as extraction, filtration, chromatography, distillation, or alternatively, can be used without isolation and/or purification in the further steps.

Typical representatives of such condensation reactions are shown in schemes (1) to (3):

(1)

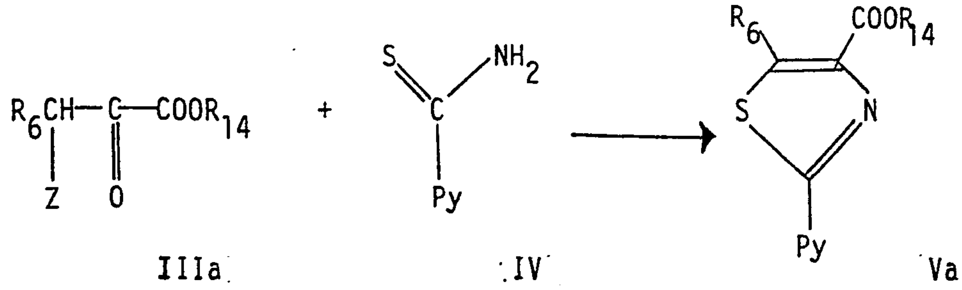

IIIa IV Va

(2)

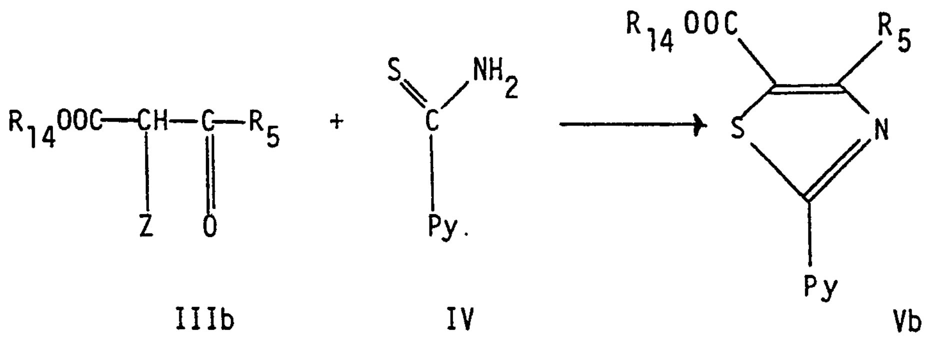

IIIb IV Vb

(3)

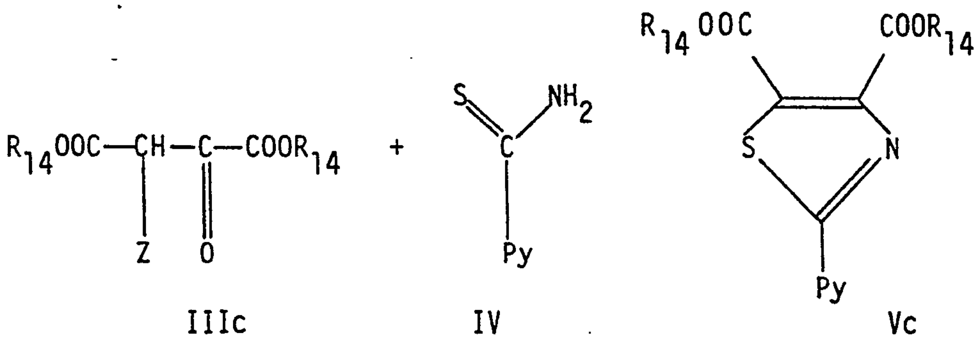

IIIc IV Vc

(The substituents $R_5$, $R_6$, $R_{14}$ and Py are as defined above).

Certain groups represented by $X_1$ and/or $X_2$, e.g. such as hydroxy, amino or substituted amino, may interfere with the condensation reaction or compounds containing such groups may be unstable as starting compounds and the result may be that a very low yield is obtained. In such cases it is preferable to either use a different process as described herein below or to use compounds of formula III which bear a different substituent and then to transfer such substituent into the desired group at a later stage.

The starting compounds of formulae III and IV are in general known compounds, as can be seen from the examples hereinafter, or they may easily be prepared by standard procedures.

The transformation of an ester group ($-COOR_{14}$) represented by $X_1$ and/or $X_2$ into the group

$-\overset{O}{\overset{\|}{C}}-NR_{11}R_{12}$ may be achieved according to standard reactions, e.g. by treating the ester with ammonia or the appropriate amine, $HNR_{11}R_{12}$.

The reaction can be accomplished by adding an excess of ammonia or of the appropriate amine to the starting compound. Preferably the reaction is carried out in the liquid phase using an inert solvent, e.g. a hydroxylic solvent or a mixture of a hydroxylic solvent and water. Alternatively one may also, instead of a solvent, use an excess of the amine. Generally the reaction is conducted at or above atmospheric pressure.

Equally, the transformation of such ester group ($-COOR_{14}$) into a group $-\overset{Y'}{\overset{//}{C}}-NHOH$ wherein Y' is O or S is easily achieved by using standard reactions, e.g. by heating the ester with hydroxylamine.

The transformation of the ester group, $-COOR_{14}$, into the group $-NR_7R_8$ implies using a more complicated reaction. Preferably the ester is first subjected to a Curtis rearrangement, e.g. as indicated below

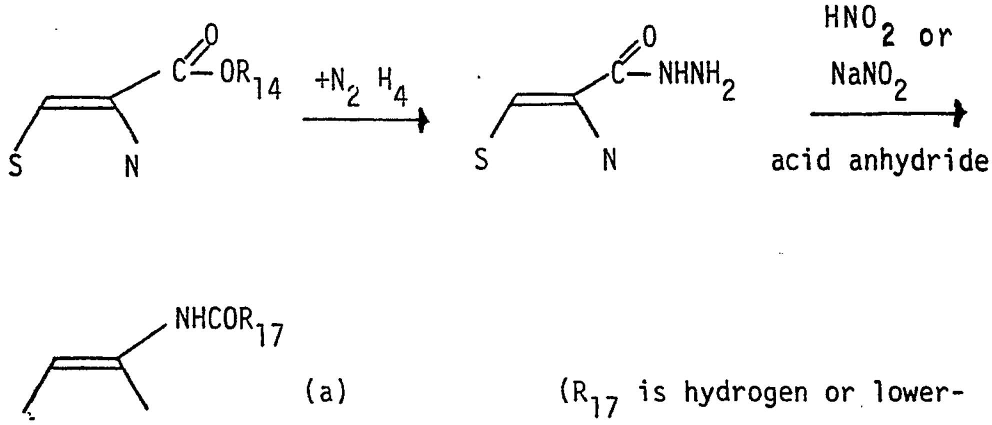


(a)

($R_{17}$ is hydrogen or lower-alkyl)

The above reaction is described in Liebigs Ann. Chem. 717, 148-153 (1968) and Heterocyclic Compounds, Thiazole and its Derivatives, Volume, 34 part 2, ppg 15-16 (John Wiley & Sons, Inc. 1979)

If the treatment with $NaNO_2$ is carried out in the presence of a mixed anhydride where one of the

acids is formic acid, then $R_{17}$ in the above compound (a) is hydrogen.

The amide (a) may easily be alkylated to a compound

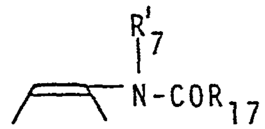 wherein $R'_7$ is loweralkyl, by first adding an essentially equimolar amount of a base, e.g. NaOH, followed by addition of an essentially equimolar amount of an alkylhalide.

The reaction is preferably carried out in an aprotic solvent such as tetrahydrofurane.

A so-obtained compound wherein $R_5$ and/or $R_6$ is replaced by the group $-\underset{R_7}{\underset{|}{N}}COR_{17}$ wherein $R_7$ and $R_{17}$ independently are hydrogen or loweralkyl is then transferred into the corresponding amine by a standard reduction procedure, e.g. by means of lithium aluminium hydride. If both $R_7$ and $R_{17}$ are hydrogen, then the reduction results in the group $-NHCH_3$. If $R_7$ is hydrogen and $R_{17}$ is loweralkyl having 1 to 5 carbon atoms, then the reduction results in a compound $-NHR_8$ wherein $R_8$ is $C_1$ to $C_6$ loweralkyl, and if $R_7$ is lower alkyl, then the reduction leads to a di-substituted amino group. An unsubstituted or mono-substituted amino group may be obtained by hydrolyzing the group $-\underset{R_7}{\underset{|}{N}}-COR_{17}$ wherein $R_7$ is hydrogen or loweralkyl.

The transformation reactions (i) to (ix) are trivial reactions well-known in the art. The alkylation of hydroxy to alkoxy [subsequent step (i) above] may be acheived according to standard procedures, e.g. by first treating the hydroxy compound with sodium hydride and thereafter with the appropriate alkylhalide.

The transformation of a group $-\overset{O}{\overset{\|}{C}}-NR_{11}R_{12}$ into $-\overset{S}{\overset{\|}{C}}-NR_{11}R_{12}$ [subsequent step(ii)] may be accomplished by standard thiation reactions e.g. by treatment of the carboxamide with $P_2S_5$ in pyridine. Any hydroxy groups present at any other position of the molecule should preferably be protected, e.g. as a benzyl ester. After completion of the reaction the protective group is removed.

The transformation of a $-\overset{O}{\overset{\|}{C}}-NH_2$ group into CN [subsequent step (iii)] proceeds easily by treating the carboxamide with p-toluene sulfonyl chloride.

The reaction may be conducted by adding approximately 2 or more equivalents of p-toluene sulfonyl chloride to the carboxamide. The reaction may be conducted in the liquid phase employing an inert organic solvent such as pyridine or dimethylformamide. Alternatively, in lieu of employing p-toluene sulfonyl chloride, an excess of phosphoryl chloride, thionylchloride, phosphorpentoxide or trifluoroacetic anhydride (in pyridine) may be employed. Reaction pressure is not critical and for

convenience the reaction is generally conducted at atmospheric pressure. The reaction is generally conducted at from 40° to 120°C., although preferably at from 80° to 110°C., and is generally complete from within 1 to 24 hours. The resulting cyano-thiazole derivative is then isolated and purified by conventional procedures such as extraction, distillation, chromatography or filtration.

The transformation of CN into $-\overset{NH}{\overset{/\!/}{C}}-NR_{11}R_{12}$ [subsequent step (iv)] may be acheived by treating the nitrile with an alkali metal alkoxide followed by treatment with an ammonium salt (e.g. $NH_4Cl$) or with the appropriate amine of the formula $HNR_{11}R_{12}$, whereby at least one of $R_{11}$ and $R_{12}$ is loweralkyl or hydroxyloweralkyl. Alternatively one may also treat the nitrile with ammonia or the appropriate amine in the presence of $NH_4Cl$ directly by heating the reactants in a steel bomb.

The reaction is known in art and is described in Journal of Organic Chemistry Vol. 27 ppg 1255-1258 (1962) and may be accomplished by treating the cyano-thiazole derivative, VIII, with liquid ammonia and ammonium chloride in a steel bomb at elevated temperatures (80°C).

Subsequent reaction (v), i.e. transformation of CN into
into $-\overset{S}{\overset{/\!/}{C}}-NH_2$, may be carried out by treating the CN-compound with $H_2S$ in the presence of triethylamine

in pyridine. The reaction is preferably carried out at or above room temperature.

The transformation of CN into $-C(=NH)-NHOH$ [subsequent step (vi)] may be accomplished by heating the nitrile with hydroxylamine hydrochloride in a mixture of water and ethanol under reflux.

The transformation of CN into $-C(=NOH)-NR_{11}R_{12}$ may be accomplished by first preparing the corresponding nitrile oxide (-CN-O) which then is treated with an amine $HNR_{11}R_{12}$. The reaction is described in Gazz. Chim. Ital., 66, 123 (1936).

The formation of the 2-imidazolyl substituent from the group CN [subsequent step (vii)] may be accomplished by first treating the nitrile with sodium methoxide, followed by reacting the so-obtained product with ethylene diamine.

The pyridyl-N-oxides of this invention [subsequent step (viii)] are readily obtained by oxidizing a compound of formula I according to standard methods, e.g. by means of hydrogen peroxide.

The reaction may be accomplished by adding an excess of the oxidizing agent, preferably 30% hydrogen peroxide (in water), to the starting pyridyl thiazole compound. The reaction may be conducted in the liquid phase employing an organic solvent which is miscible with water such as acetic acid. Reaction pressure is not critical and for convenience the reaction is generally conducted

under atmospheric pressure at from 50° to 100°C. and is generally complete from within 1 to 24 hours. The N-oxide is then isolated and purified by conventional procedures such as distillation, filtration, chromatography, extraction and the like.

The compounds of this invention easily form acid addition salts. The salts may be prepared by mixing a compound of formula I or a suitable derivative thereof with the acid or derivative thereof, e.g. a salt. Quite frequently, however, depending on the reagents used, the immediate product obtained by one or the other of the reactions described in the examples will be an acid addition salt.

B: A further basic concept for preparing the compounds of this invention is characterized in that a compound of the general formula IV

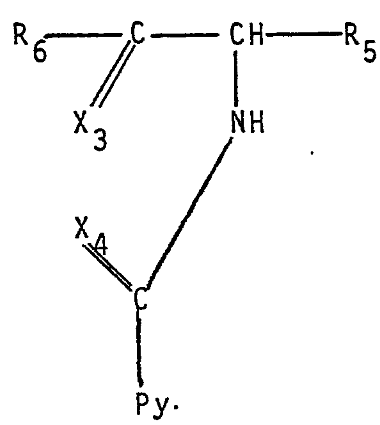

VI

wherein Py, $R_5$ and $R_6$ are as above defined and one of $X_3$ and $X_4$ is S and the other is O, is subjected to an intramolecular condensation.

The starting compounds of formula VI are either known compounds (e.g. as shown in the examples) or may be obtained by standard methods well-known in the art.

Obviously the condensation may proceed more or less easily, depending on the groups represented by $R_5$ and $R_6$. Also the one or the other of the compounds of formula VI with specific $R_5$ and/or $R_6$ groups may be difficult to prepare. In such instances one would preferably first prepare compounds of formula I having different $R_5$ and/or $R_6$ substitutents and then subsequently transfer such other substituent into the desired $R_5$ and/or $R_6$. Obviously certain of the subsequent reactions (i) to (ix) listed under process A above, may also be carried out after reaction B has been completed.

Typical reactions according to the above procedure are exemplified by the reaction schemes (4) and (5):

(4)

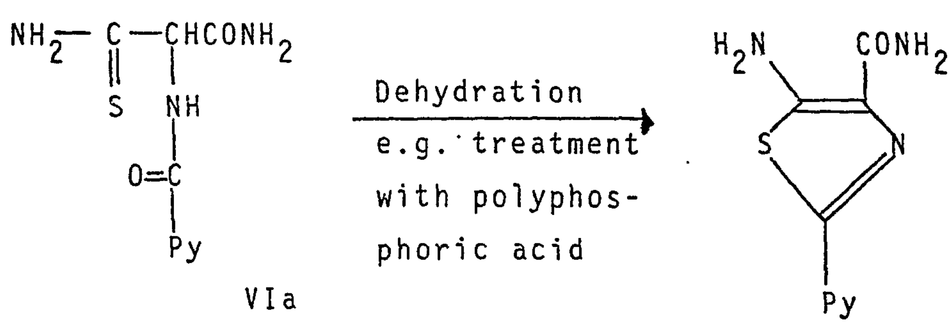


VIa

(5) 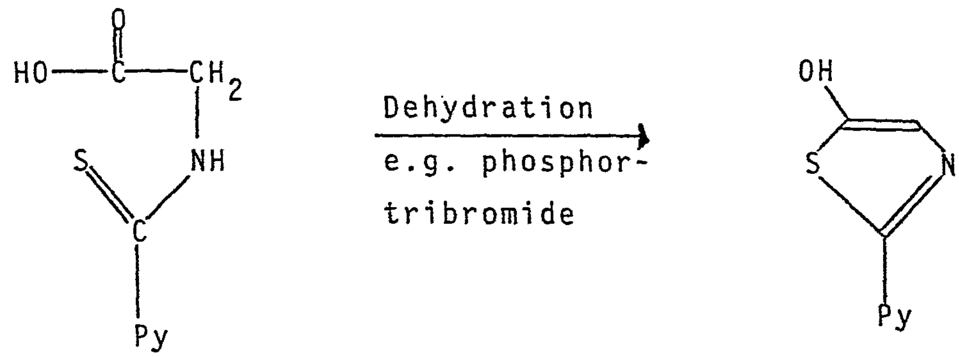


VIb

These intramolecular condensation reactions are standard dehydration reactions involving heating the starting compound with a suitable dehydrating agent, e.g. polyphosphoric acid, trifluoroacetic acid or phosphortribromide etc.

Reaction (5) is described in Heterocyclic Compounds, Thiazole and Its Derivatives, Volume 34 part 2, ppg. 426-428 (John Wiley and Sons, Ind. 1979).

Reaction (4) is described in Japanese Patent No. 7.103.972 (reported in Chemical Abstracts 74, 141756r, 1971).

As is obvious to anyone skilled in the art, there are numerous further reactions which may permit changing one of the substituents $R_1$ to $R_6$ into other substituents. Thus, for example, a compound wherein $R_1$,$R_2$,$R_3$ and/or $R_4$ is halogen may be converted into a compound wherein one or more of the substituents represent hydroxy or amino by standard reactions.

## EXAMPLE 1

## PREPARATION OF 4-HYDROXY-2-(4-PYRIDYL)-THIAZOLE HYDROBROMIDE

Add 6.95 gm of bromoacetic acid to 70 ml of toluene along with 6.90 gm of thioisonicotinamide. Heat the system at reflux for 6 hours. Stop the reaction and cool the system to room temperature. Decant the toluene from the residue. Triturate the residue with absolute ethanol and filter to yield 4-hydroxy-2-(4-pyridyl)-thiazole hydrobromide.

(m.p. $>$ 290°C.)

## EXAMPLE 2

## PREPARATION OF 4-HYDROXY-2-(4-PYRIDYL)-THIAZOLE-5-CARBOXAMIDE

(a) Add 58.1 gm of ethyl bromomalonate to a suspension of 32.5 gm of thioisonicotinamide in 200 ml of absolute ethanol. Stir the system at room temperature for 72 hours. Stop the reaction and filter the solid. Recrystallize the solid from aqueous ethanol to yield 4-hydroxy-2-(4-pyridyl)-thiazole-5-carboxylate.

(b) Add 22.0 gm of 4-hydroxy-2-(4-pyridyl)-thiazole-5-carboxylate to 300 ml of 20% ammonia in methanol in a steel bomb cooled to 0° to 5°C. Heat the system in an oil bath to 80° C. for 70 hours. Stop the reaction and cool the system to room temperature. Remove the solvent by stripping to yield a solid residue. Crystallize the residue from aqueous ethanol to give 4-hydroxy-2-(4-pyridyl)-thiazole-5-carboxamide.

(m.p. $>$ 230°C.)

## EXAMPLE 3

## PREPARATION OF 2-(4-PYRIDYL)-THIAZOLE-5-CARBOXAMIDE

(a) Add 18.0 gm of methyl 2-chloroformylacetate to 18.2 gm of thioisonicotinamide in 250 ml of methanol. Heat the system to reflux for 22 hours. Stop the reaction and cool the system in an ice bath. Filter the solution to yield methyl 2-(4-pyridyl)-thiazole-5-carboxylate.

(m.p. 163° - 165°C.)

(b) Add 10.0 gm of methyl 2-(4-pyridyl)-thiazole-5-carboxylate to 200 ml of 20% ammonia in methanol in a steel bomb cooled to 0° to 5°C. Heat the system in an oil bath to 80°C for 70 hours. Stop the reaction and cool the system to room temperature. Remove the solvent by stripping to yield a solid residue. Crystallize the residue from aqueous ethanol to give 2-(4-pyridyl)-thiazole-5-carboxamide.

(m.p. 267° - 270°C.)

## EXAMPLE 4

## PREPARATION OF 4-METHYL-2-(4-PYRIDYL)-THIAZOLE-5-CARBOXAMIDE

(a) Add 10.3 gm of ethyl 2-chloroacetoacetate to 75 ml of methanol along with 17.3 gm of thioisonicotinamide. Heat the system to reflux for 4 hours. Stop the reaction and remove the solvent by stripping to yield a solid residue. Add 200 ml of toluene to the residue. Filter the solution and extract the filtrate first with 1N sodium hydroxide solution and then with 6N hydrochloric acid. Add concentrated ammonium hydroxide

solution to the acid extract until the system is basic. Extract the product from this solution with chloroform. Dry the chloroform over anhydrous sodium sulfate and then filter the solution. Remove the chloroform by stripping to give a solid residue. Recrystallize the solid residue from 2-propanol to yield ethyl 4-methyl-2-(4-pyridyl)-thiazole-5-carboxylate.

(b) Add 25.0 gm of ethyl 4-methyl-2-(4-pyridyl)-thiazole-5-carboxylate to 300 ml of 20% ammonia in methanol in a steel bomb cooled to 0° to 5°C. Heat the system in an oil bath to 80°C for 70 hours. Stop the reaction and remove the solvent by stripping to yield a solid. Recrystallize the solid from aqueous ethanol to give the 4-methyl-2-(4-pyridyl)-thiazole-5-carboxamide.

(m.p. 244° - 247°C., dec.)

EXAMPLE 5

PREPARATION OF 2-(4-PYRIDYL)-THIAZOLE-4-CARBOXAMIDE

(a) Add 70.0 gm of ethyl bromopyruvate to 41.5 gm of thioisonicotinamide in 600 ml of absolute ethanol. Heat the system at reflux for 2 1/2 hours. Stop the reaction and cool the system to room temperature. Filter off the solid and recrystallize the solid from absolute ethanol to give the ehtyl 2-(4-pyridyl)-thiazole-4-carboxylate hydrobromide•1/4 hydrate.

(m.p. 217° - 222°C.)

(b) Add 25.0 gm of ethyl 2-(4-pyridyl)-thiazole-4-carboxylate hydrobromide 1/4 hydrate to 300 ml of 20% ammonia in methanol in a steel bomb cooled to 0° to 5°C. Heat the system in an oil bath at 65°C. for 48 hours. Stop the reaction and cool the system to room temperature. Remove the solvent by stripping to yield a solid residue. Crystallize the residue from aqueous ethanol to give the 2-(4-pyridyl)-thiazole-4-carboxamide.

(m.p. 201° - 204.5°C.)

EXAMPLE 6

PREPARATION OF N-METHYL-2(4-PYRIDYL)-THIAZOLE-5-CARBOXAMIDE

Add 5.5 gm of ethyl 2-(4-pyridyl)-thiazole-4-carboxylate hydrobromide•1/4 hydrate to a steel bomb containing 40% aqueous methylamine. Heat the system in an oil bath at 100°C. for 16 hours. Stop the reaction and cool the system to room temperature. Extract the product with chloroform and wash the chloroform solution with water. Treat the chloroform solution with anhydrous sodium sulfate and filter. Remove the solvent by stripping to give the N-methyl-2-(4-pyridyl)-thiazole-4-carboxamide.

(m.p. 155° - 158.5°C.)

EXAMPLE 7

PREPARATION OF 5-HYDROXY-2-(4-PYRIDYL)-THIAZOLE

Add 2 gm of N-thiocarbonyl-4-pyridyl glycine to phosphorus tribromide. Stir the solution at room temperature until the reaction is complete. Stop the reaction and isolate 5-hydroxy-2-(4-pyridyl)-thiazole.

(m.p.

## EXAMPLE 8

## PREPARATION OF 4-ETHOXY-2-(4-PYRIDYL)-THIAZOLE

Add 4.8 gm of sodium hydride (50% by weight in oil) to 1.77 gm 4-hydroxy-2-(4-pyridyl)-thiazole in 100 ml of dimethylformamide. Heat the system to 110°C. for 4 hours. Cool the system to room temperature. Add 1.56 gm of ethyl iodide to the system. Stir the system at room temperature for 3 hours. Filter the solution and remove the solvent by stripping to give 4-ethoxy-2-(4-pyridyl)-thiazole.

(m.p.

## EXAMPLE 9

## N-(2-HYDROXYETHYL)-2-(4-PYRIDYL)-THIAZOLE-4-CARBOXAMIDE 1/4 HYDRATE

Add 3.5 gm of ethyl 2-(4-pyridyl)-thiazole-4-carboxylate hydrobromide 1/4 hydrate to a steel bomb containing 5 ml of ethanolamine in 75 ml of ethanol. Heat the system in an oil bath at 100°C. for 20 hours. Cool the system to room temperature and remove the solvent by stripping. Partition the residue between chloroform and water. Dry the chloroform solution over anhydrous magnesium sulfate, filter and remove the solvent by stripping. Crystallize the residue from aqueous ethanol to give N-(2-hydroxyethyl)-2-(4-pyridyl)-thiazole-4-carboxamide 1/4 hydrate.

(m.p. 159° - 162°C.)

## EXAMPLE 10

## PREPARATION OF 4-CYANO-2-(4-PYRIDYL)-THIAZOLE

Add 10.8 gm of p-toluenesulfonyl chloride to 5.20 gm of 2-(4-pyridyl)-thiazole-4-carboxamide in 100 ml of pyridine cooled to 0° to 5°C. Heat the system to 90°C. in an oil bath for 18 hours. Remove the solvent by stripping and dissolve the residue in 200 ml of 1N hydrochloric acid. Extract the acid solution with chloroform. Cool the chloroform solution to 0° to 5°C. and then add 1N sodium hydroxide solution until the system is alkaline. Extract the product from this solution with chloroform. Wash the chloroform solution with water and then dry the chloroform solution with anhydrous magnesium sulfate. Filter the solution and remove the solvent by stripping to give the 4-cyano-2-(4-pyridyl)-thiazole.

(m.p. 181.5° - 182.5°C.)

## EXAMPLE 11

## PREPARATION OF 4-METHYL-5-CYANO-2-(4-PYRIDYL)-THIAZOLE

Add 10.0 gm of 4-methyl-2-(4-pyridyl)-thiazole-5-carboxamide to 100 ml of phosphoryl chloride. Heat the system at reflux for 6 hours. Remove the phosphoryl chloride by distillation at reduced pressure. Cool the residue to room temperature and then treat the residue with 300 ml 5% sodium bicarbonate solution. Collect the resulting precipitate by filtration and dissolve the solid in 500 ml of chloroform. Treat the solution with charcoal and dry the solution over anhydrous magnesium sulfate. Filter and remove the solvent by stripping to give the 4-methyl-5-cyano-2-(4-pyridyl)-thiazole.

(m.p. )

Similarly, by following the same procedures as outlined in Examples 10 and 11 above and employing the appropriate reagents, the following compounds can be prepared:

5-cyano-2-(4-pyridyl)-thiazole;
5-ethyl-4-cyano-2-(4-pyridyl)-thiazole.

EXAMPLE 12

PREPARATION OF 4-AMINO-2-(4-PYRIDYL)-THIAZOLE

(a) Add 5.9 gm of ethyl 2-(4-pyridyl)-thiazole-4-carboxylate to 100 ml of absolute ethanol along with 10 ml of 99-100% hydrazine hydrate. Stir the system at reflux for 16 hours. Remove the solvent by stripping to give a solid residue. Add 100 ml of chloroform to the residue and stir overnight. Filter the product from the solution. Dissolve the product in isopropanol and treat the system with charcoal. Filter and recrystallize the product from isopropanol to give 2-(4-pyridyl)-thiazole-4-carbohydrazide.

(b) Add 2.20 gm of 2-(4-pyridyl)-thiazole-4-carbohydrazide to 30 ml of 2N hydrochloric acid at 5°C. Add 2.07 gm of sodium nitrite in portions keeping the temperature at 5°C. Stir for 1/2 hour after addition of the sodium nitrite. Filter the solution and dry the solid over phosphorus pentoxide at reduced pressure. Add the solid azide to 6 ml of a 5:1 mixture of acetic anhydride/acetic acid. Heat the system to 95°C. and stir for 1/2 hour. Cool to 0°C. and then add water. Neutralize the solution with sodium carbonate. Filter the resulting suspension to give N-acetyl 4-amino-2-(4-pyridyl)-thiazole. Recrystallize this product from

aqueous ethanol to give the pure compound.

(c) Add 1.21 gm of N-acetyl 4-amino-2-(4-pyridyl)-thiazole to 60 ml of a 1:1 solution of 15% sodium hydroxide (in water)/methanol. Heat the system at reflux until the reaction is completed. Remove the methanol by stripping. Extract the product with chloroform from the aqueous solution. Dry the chloroform solution over anhydrous magnesium sulfate. Filter the solution and remove the chloroform by stripping to give 4-amino-2-(4-pyridyl)-thiazole.

(m.p. )

## EXAMPLE 13

## PREPARATION OF N-ETHYL-4-AMINO-2-(4-PYRIDYL)-THIAZOLE

Add 2.37 gm of N-acetyl 4-amino-2-(4-pyridyl)-thiazole hydrate to 100 ml of diethyl ether. Cool the system to 0° to 5°C. and then add 0.38 gm of lithium aluminum hydride to the system. Heat the system to 35°C. and stir at 35°C. for 4 hours. Stop the reaction isolate 4-(N-ethylamino)-2-(4-pyridyl)-thiazole.

(m.p. )

EXAMPLE 14

PREPARATION OF 2-(4-PYRIDYL)-THIAZOLE-4-CARBOXAMIDINE HYDROCHLORIDE

Add 1.87 gm of 4-cyano-2-(4-pyridyl)-thiazole to 0.53 gm ammonium chloride in liquid ammonia in a steel bomb. Heat the system to 80°C. for 24 hours. Cool the system to room temperature and remove the ammonia. Isolate the 2-(4-pyridyl)-thiazole-4-carboxamidine hydrochloride.

(m.p. 340°C., dec.)

EXAMPLE 15

2-(4-PYRIDYL)-THIAZOLE-4-CARBOXAMIDINE HYDROCHLORIDE

Add 0.30 gm of sodium methoxide to 3.73 gm of 4-cyano-2-(4-pyridyl)-thiazole in 400 ml of methanol and stir under nitrogen at room temperature for 16 hours. Add 1.08 gm of ammonium chloride and heat at reflux for 4 huurs. Remove one-half the solvent by distillation. Cool in an ice bath and filter to give 2-(4-pyridyl)-thiazole-4-carboxamidine hydrochloride.

(m.p. 340°C., dec.)

EXAMPLE 16

PREPARATION OF 2-(4-PYRIDYL)-THIAZOLE-4-THIOCARBOXAMIDE

Dissolve 2.0 gm of 4-cyano-2-(4-pyridyl)-thiazole in 25.0 gm of pyridine. Add 1.5 ml of triethylamine to the system. Cool the system to 0° to 6°C. and treat the system with hydrogen sulfide gas until the reaction is complete as indicated by thin layer chromatography.

Pour the reaction mixture into icewater and filter to give 2-(4-pyridyl)-thiazole-4-thiocarboxamide.

(m.p. 178° - 180°C.)

EXAMPLE 17

2-(4-PYRIDYL)-4-(2-IMIDAZOLIN-2-YL)-THIAZOLE

Add 0.18 gm of sodium methoxide to 1.75 gm of 4-cyano-2-(4-pyridyl)-thiazole in 300 ml of methanol and stir overnight under nitrogen at room temperature. Remove the solvent by stripping. Partition the residue between cold water and cold chloroform. Dry the chloroform solution over anhydrous sodium sulfate, filter and remove the solvent by stripping at room temperature. Add 50 ml of absolute ethanol and 1.56 ml of ethylene-diamine and heat at 60° to 70°C. for 52 hours. Remove the solvent by stripping and partition the residue between water and chloroform. Wash the chloroform solution with water and dry over anhydrous magnesium sulfate. Filter and remove the solvent by stripping to give a solid residue. Recrystallize the product from benzene to give 2-(4-pyridyl)-4-(2-imidazolin-2-yl)-thiazole.

(m.p. 139° - 141°C.)

EXAMPLE 18

2-(4-PYRIDYL)-THIAZOLE-4-CARBOXAMIDOXIME HYDROCHLORIDE

Add a solution of 0.56 gm of hydroxylamine hydrochloride in 25 ml of water to a solution of 1.50 gm of 4-cyano-2-(4-pyridyl)-thiazole in 300 ml of warm ethanol. Heat at reflux until the reaction is complete as indicated by thin layer chromotography on silica gel eluted with

ethyl acetate. Cool the reaction mixture in an ice bath and filter the resulting suspension to give 2-(4-pyridyl)-thiazole-4-carboxamidoxime hydrochloride.

(m.p. 258° - 260°C. dec.)

EXAMPLE 19

PREPARATION OF 2-(4-PYRIDYL)-THIAZOLE-4-CARBOXAMIDE-N-OXIDE

Add 1.42 ml of 30% hydrogen peroxide to 0.50 gm of 2-(4-pyridyl)-thiazole-4-carboxamide in 10 ml of glacial acetic acid at room temperature. Heat the system in an oil bath at 85°C. Add additional aliquots (1.0 ml) of 30% hydrogen peroxide until the reaction is complete as indicated by thin layer chromatography on silica gel eluted with ethyl acetate. Cool the system to room temperature and then add aqueous ethanol to give a suspension. Collect the product by filtration and air dry to give the title compound.

(m.p. 301° -304°C.)

EXAMPLE 20

2-(4-PYRIDYL)-THIAZOLE-4,5-DICARBOXAMIDE

(a) Add 26.7 gm of diethyl α-chloro-β-ketosuccinate to 13.6 gm of thioisonicotinamide in 250 ml of absolute ethanol. Heat at reflux for 18 hours. Cool the system in an ice bath and filter to give diethyl 2-(4-pyridyl)-thiazole-4,5-dicarboxylate hydrochloride.

(b) Add the diester from part (a) above to 300 ml of 20% ammonia in methanol in a steel bomb cooled to

0° to 5°C. Heat the system in an oil bath at 65°C. for 48 hours. Cool the system to room temperature and remove the solvent by stripping. Crystallize the residue from aqueous ethanol to give 2-(4-pyridyl)-thiazole-4,5-dicarboxamide.

(m.p. )

EXAMPLE 21

4-CYANO-2-(4-PYRIDYL)-THIAZOLE-5-CARBOXAMIDE AND 5-CYANO-2-(4-PYRIDYL) THIAZOLE-4-CARBOXAMIDE

Add 24.8 gm of 2-(4-pyridyl)-thiazole-4,5-dicarboxamide to 500 ml of dioxane containing 16.0 ml of pyridine. Add 14.1 ml of trifluoroacetic anhydride dropwise with stirring with the temperature maintaining below 10°C. by cooling. Stir at 10°C. for 2 hours, then for 18 hours at room temperature. Remove the solvent by stripping and treat the residue with 400 ml fo 5% sodium bicarbonate solution. Chromatograph the resulting precipitate on silica gel to give 4-cyano-2-(4-pyridyl)-thiazole-5-carboxamide and 5-cyano-2-(4-pyridyl)-thiazole-4-carboxamide.

(m.p. )

EXAMPLE 22

4-CARBAMOYL-2-(4-PYRIDYL)-THIAZOLE-5-CARBOXAMIDINE HYDROCHLORIDE

Add 2.3 gm of 5-cyano-2-(4-pyridyl)-thiazole-4-carboxamide to a solution of 0.54 gm of sodium methoxide in 400 ml of methanol under nitrogen and stir at room temperature for 16 hours. Add 1.1 gm of ammonium

chloride and heat at reflux for two hours. Remove one-half of the solvent by distillation. Cool in an ice bath and filter to give 4-carbamoyl-2-(4-pyridyl)-thiazole-5-carboxamidine hydrochloride.

(m.p. )

EXAMPLE 23

5-AMINO-2-(4-PYRIDYL)-THIAZOLE-4-CARBOXAMIDE

Following the procedure described in Japanese Patent 7,103,972 (Chemical Abstracts 74, 141756r, 1971) heat N-isonicotinyl aminomalonthioamide with polyphosphoric acid at 120°C. for 2 hours. Cool and treat the reaction with 5% aqueous sodium bicarbonate to give 5-amino-2-(4-pyridyl)-thiazole-4-carboxamide.

(m.p. )

Typical representatives of compounds of this invention are compiled in the following table.

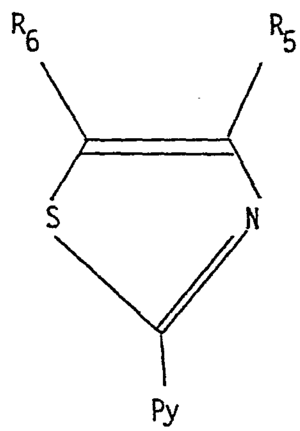

| Py | $R_5$ | $R_6$ | Salt or Hydrate | m.p.°C. |
|---|---|---|---|---|
| 4-pyridyl | $-\overset{O}{\overset{\|\|}{C}}-NH_2$ | H | - | 201-204.5 |
| 4-pyridyl | CN | H | – | 181.5-182.5 |
| 4-pyridyl | -OH | $-\overset{O}{\overset{\|\|}{C}}-NH_2$ | $1/4H_2O$ | $>230$ |
| 4-pyridyl | $CH_3$ | $-\overset{O}{\overset{\|\|}{C}}-NH_2$ | – | 244-247 (dec.) |
| 4-pyridyl | OH | H | HBr | $>290$ |
| 4-pyridyl | $-\overset{O}{\overset{\|\|}{C}}-NHCH_3$ | H | – | 155-158.5 |
| 4-pyridyl-N-oxide | $-\overset{O}{\overset{\|\|}{C}}-NH_2$ | H | – | 301-304 |

| Py | $R_5$ | $R_6$ | Salt or Hydrate | m.p.°C. |
|---|---|---|---|---|
| 4-pyridyl | H | 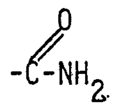 | — | 267-270 |
| 4-pyridyl | 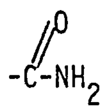 | $CH_3$ | — | 178-182 |
| 4-pyridyl | 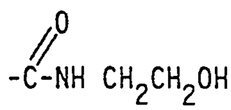 | H | 1/4 $H_2O$ | 159-162 |
| 4-pyridyl | $-NH_2$ | H | 1/3 $H_2O$ | 153-157 |
| 4-pyridyl | 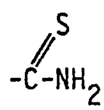 | H | — | 178-180 |
| 4-pyridyl | 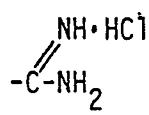 | H | HCl | 340(dec.) |
| 2-methyl-4-pyridyl | 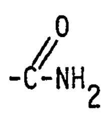 | H | — | 220-222.5 |
| 4-pyridyl | 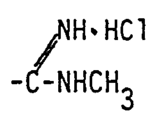 | H | HCl | 345-348 (dec.) |
| 4-pyridyl | 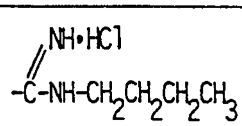 | H | HCl 1/2 $H_2O$ | 162 |
| 4-pyridyl | 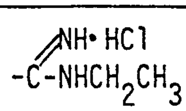 | H | HCl 3/4 $H_2O$ | 264-267 (dec.) |

| Py | $R_5$ | $R_6$ | Salt or Hydrate | m.p.°C. |
|---|---|---|---|---|
| 4-pyridyl | 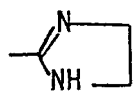 | H | - | 139-141 |
| 4-pyridyl | 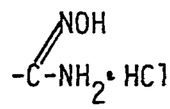 | H | HCl | 258-260 (dec.) |
| 4-pyridyl | 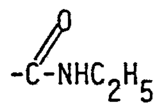 | H | - | |
| 4-pyridyl | H | 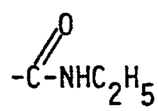 | - | |
| 4-pyridyl | H | 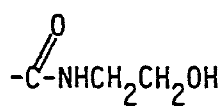 | - | |
| 4-pyridyl | H | CN | - | |
| 4-pyridyl | H | 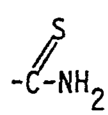 | - | |
| 4-pyridyl | H | 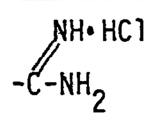 | HCl | |
| 4-pyridyl | H | 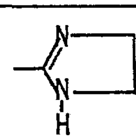 | - | |
| 4-pyridyl | H | 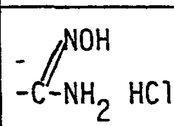 | HCl | |

4-methyl-5-cyano-2-(4-pyridyl)-thiazole;
N-methyl-4-amino-2-(4-pyridyl)-thiazole;
4-ethoxy-2-(4-pyridyl)-thiazole;
2-(3-amino-4-pyridyl)-thiazole-5-carboxamide;
4-ethyl-2-(4-pyridyl)-thiazole-5-thio-carboxamide;
N-methyl-2-(2-methyl-6-bromo-4-pyridyl)-thiazole-5-carboxamide;
5-methyl-4-trifluoromethyl-2-(2-n-propyl-4-pyridyl)-thiazole;
5-ethyl-4-trifluoromethyl-2-(2-methyl-3-chloro-4-pyridyl)-thiazole;
4-hydroxy-2-(2-methoxy-4-pyridyl)-thiazole;
4-hydroxy-2-(2-n-propoxy-4-pyridyl)-thiazole;
4-hydroxy-2-(2-hydroxy-3-bromo-4-pyridyl)-thiazole;
5-hydroxy-2-(3-hydroxy-4-pyridyl)-thiazole;
N-ethyl 4-ethyl-2-(4-pyridyl)-thiazole-5-carboxamide;
N-ethyl 4-n-propyl-2-(4-pyridyl)-thiazole-5-carboxamide;
N-methyl 4 methyl-2-(4-pyridyl)-thiazole-5-carboxamide;
N-n-propyl 5-methyl-2-(4-pyridyl)-thiazole-4-carboxamide;
N-n-hexyl 5-ethyl-2-(4-pyridyl)-thiazole-4-carboxamide;
N-methyl 5-n-propyl-2-(4-pyridyl)-thiazole-4-carboxamide;
N,N-diethyl 5-propoxy-2-(4-pyridyl)-thiazole-4-carboxamide;
N,N-dimethyl 5-methoxy-2-(4-pyridyl)-thiazole-4-carboxamide;
N-ethyl N-methyl 5-cyano-2-(2,3-dimethyl-4-pyridyl)-thiazole-4-carboxamide;
N-isopropyl 5-cyano-2-(3-ethyl-4-pyridyl)-thiazole-4-carboxamide;

N-ethyl 5-(2-hydroxyethyl)-2-(4-pyridyl)-thiazole-4-carboxamide;

N-methyl 5-methyl-2-(4-pyridyl)-thiazole-4-carboxamide;

N,N-dimethyl 5-methyl-2-(3-methyl-4-pyridyl)-thiazole-4-carboxamide;

N,N-diethyl 2-(3-methyl-4-pyridyl)-thiazole-4-carboxamide;

N-n-hexyl-N-ethyl 2-(4-pyridyl)-thiazole-5-carboxamide;

N-isopropyl-N-ethyl 4-ethyl-2-(4-pyridyl)-thiazole-5-carboxamide;

N-ethyl 4-n-propyl-2-(4-pyridyl)-thiazole-5-carboxamide;

N-n-propyl 5-methyl-2-(4-pyridyl)-thiazole-4-carboxamide;

N-n-hexyl 5-ethyl-2-(4-pyridyl)-thiazole-4-carboxamide;

N-ethyl 5-cyano-2-(4-pyridyl)-thiazole-4-carboxamide;

N-ethyl 4-hydroxy-2-(4-pyridyl)-thiazole-5-carboxamide;

5-hydroxy-4-methyl-2-(4-pyridyl)-thiazole

N-methyl 5-methyl-2-(4-pyridyl)-thiazole-4-carboxamide;

N,N-dimethyl 5-methyl-2-(4-pyridyl)-thiazole-4-carboxamide;

N-ethyl 5-cyano-2-(4-pyridyl)-thiazole-4-carboxamide;

N-(2-hydroxyethyl) 5-methoxy-2-(4-pyridyl)-thiazole-4-carboxamide;

N-n-propyl 5-cyano-2-(3-ethyl-4-pyridyl)-thiazole-4-carboxamide;

N,N-di-n-propyl 5-methyl-2-(4-pyridyl)-thiazole-4-carboxamide;

5-ethyl-4-cyano-2-(4-pyridyl)-thiazole;

4-hydroxy-2-(2-methoxy-4-pyridyl-N-oxide)-thiazole;

5-hydroxy-2-(3-hydroxy-4-pyridyl-N-oxide)-thiazole;

5-hydroxy-4-methyl-2-(4-pyridyl-N-oxide)-thiazole;

5-ethyl-4-trifluoromethyl-2-(2-methyl-3-chloro-4-pyridyl-N-oxide)-thiazole;

N,N-diethyl-5-amino-2-(4-pyridyl)-thiazole;

N,N-di-n-propyl-5-amino-4-methyl-2-(4-pyridyl)-thiazole;

4-amino-2-(3-chloro-4-pyridyl)-thiazole;

5-amino-4-ethoxy-2-(4-pyridyl)-thiazole;

4-amino-5-ethoxy-2-(4-pyridyl)-thiazole;

5-amino-4-methyl-2-(4-pyridyl)-thiazole;

2-(4-pyridyl)-thiazole-5-carboxamidine hydrochloride;

4-methyl-2-(4-pyridyl)-thiazole-5-carboxamidine hydrochloride;

4-ethyl-2-(4-pyridyl)-thiazole-5-carboxamidine hydrochloride;

5-ethyl-2-(4-pyridyl)-thiazole-4-carboxamidine hydrochloride;

5-methyl-2-(4-pyridyl)-thiazole-4-thiocarboxamide;

5-methoxy-2-(3-chloro-4-pyridyl)-thiazole-4-thiocarboxamide;

2-(4-pyridyl)-thiazole-5-thiocarboxamide; and

2-(4-pyridyl-N-oxide)-thiazole-4-carboxamide.

The compounds of this invention have been found to increase cardiac contractility. As such, the compounds of this invention are useful in the treatment of congestive heart failure.

The compounds were tested for their ability to increase cardiac contractility both *in vitro* and *in vivo*.

*In vitro* tests were conducted on left atria obtained from guinea pigs employing the method described in Am. J. Physiology 221:1470-1475 (1971). Increases in cardiac contractility were found at concentrations ranging from 1 µgm/ml to 1000µgm/ml with increases in cardiac contractility found generally from 10 µgm/ml to 100 µgm/ml.

*In vivo* tests were conducted on open-chest barbituated-anaesthetised dogs employing the method described in J. Pharmacology Exp. Ther. 218, 442-452 (1982). Increases in cardiac contractility were generally found at dosage levels of 1 mg/kg to 10 mg/kg.

Typically, a daily dosage regimen would generally be 0.1 to 1 gm/day oral and 0.05 to 0.5 gm/day parenterally. The actual dose to be administered is determined by the clinician and is dependent upon various factors such as the particular compound employed, age and weight of the subject the severity of the disease and the individual response.

The compounds are preferably administered either orally or parenterally with the preferred mode of administration dependent on the severity of the individual's condition.

For example with an individual suffering an acute case of congestive heart failure the preferred mode of administration would be parenterally while for a chronic case of heart failure, the preferred mode of administration would be orally. Also transdermal application is conceivable.

Besides, as mentioned above, compounds of this invention, i.e. those of formula II, can be used to treat allergy caused diseases and their preferred use is for treating allergic chronic obstructive lung diseases. Chronic obstructive lung disease as used herein means disease conditions in which the passage of air through the lungs is obstructed or diminished such as is the case in asthma, bronchitis and the like.

The bronchodilating effect of the compounds of this invention is identified by tests which measure a compound's inhibition of anaphylatic bronchospasm in sensitized guinea pigs having antigen induced bronchoconstriction. For example, the compound 2-(4-pyridyl)-thiazole-4-thiocarboxamide was found to inhibt anaphylactic bronchospasms in such test procedure when given at an intravenous dose of 10 mg/kg. The compounds are effective non-adrenergic, antianaphylactic agents. When administered parenterally, e.g., intravenously, the compounds are active at dosages from about 0.1-10 mg/kg body weight.

For preparing pharmaceutical compositions from the compounds described by this invention, inert, pharmaceutically acceptable carriers can be either solid or liquid. Solid form preparations include powders, tablets, dispersible granules, capsules, cachets and suppositories.

A solid carrier can be one or more substances which may also act as diluents, flavouring agents, solubilizers, lubricants, suspending agents, binders or tablet disintegrating agents; it can also be an encapsulating material. In powders, the carrier is a finely divided solid which is in admixture with the finely divided active compound. In the tablet the active compound is mixed with carrier having the necessary binding properties in suitable proportions and compacted in the shape and size desired. The powders and tablets preferably contain from 5 or 10 to about 70 percent of the active ingredient. Suitable solid carriers are magnesium carbonate, magnesium stearate, talc, sugar, lactose, pectin, dextrin, starch, gelatin, tragacanth, methylcellulose, sodium carboxymethylcellulose, a low melting wax, cocoa butter and the like. The term "preparation" is intended to include the formulation of the active compound with encapsulating material as carrier providing a capsule in which the active component (with or without other carriers) is surrounded by carrier, which is thus in association with it. Similarly, cachets are included. Tablets, powders, cachets and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized moulds, allowed to cool and thereby solidify.

Liquid form preparations include solutions, suspensions and emulsions. As an example may be mentioned water or water-propylene glycol solutions for parenteral injection. Liquid preparations can also be formulated in solution in aqueous polyethylene glycol solution. Aqueous solutions suitable for oral use can be prepared by adding the active component in water and adding suitable colourants, flavours, stabilizing, sweetening, solubilizing and thickening agents as desired. Aqueous suspension suitable for oral use can be made by dispersing the finely divided active component in water with viscous material, i.e., natural or synthetic gums, resins, methylcellulose, sodium carboxymethylcellulose and other well-known suspending agents.

Also included are solid form preparations which are intended to be converted , shortly before use, to liquid form preparations for either oral or parenteral administration. Such liquid forms include solutions, suspensions and emulsions. These particular solid form preparations are most conveniently provided in unit dose form and as such are used to provide a single liquid dosage unit. Alternately, sufficient solid may be provided so that after conversion to liquid form, multiple individual liquid doses may be obtained by measuring pre-determined volumes of the liquid form preparation as with a syringe, teaspoon or other volumetric container. When multiple liquid doses are so prepared, it is preferred to maintain the unused portion of said liquid doses at low temperature (i.e., under refrigeration) in order to retard possible decomposition. The solid form preparations intended to be converted to liquid form may contain, in addition to the active material, flavourants, colourants, stabilizers,

buffers, artificial and natural sweetners, dispersants, thickeners, solubilizing agents and the like. The solvent utilized for preparing the liquid form preparation may be water, isotonic water, ethanol, glycerine, propylene glycol and the like as well as mixtures thereof. Naturally, the solvent utilized will be chosen with regard to the route of administration, for example, liquid preparations containing large amounts of ethanol are not suitable for parenteral use.

Preferably, the pharmaceutical preparation is in unit dosage form. In such form, the preparation is subdivided into unit doses containing appropriate quantities of the active component. The unit dosage form can be a packaged preparation, the package containing discrete quantities of preparation, for example, packeted tablets, capsules and powders in vials or ampoules. The unit dosage form can also be a capsule, cachet or tablet itself or it can be the appropriate number of any of these in packaged form.

The quantity of active compound in a unit dose of preparation may be varied or adjusted from 1 mg to 100 mg according to the particular application and the potency of the active ingredient. The compositions can, if desired, also contain other therapeutic agents.

The dosages may be varied depending upon the requirements of the patient, the severity of the condition being treated and the particular compound being employed. Determination of the proper dosage for a particular situation is within the skill of the art. Generally, treatment is initiated with smaller dosages which are less than the optimum dose of the compound.

Thereafter, the dosage is increased by small increments until the optimum effect under the circumstances is reached. For convenience, the total daily dosage may be divided and administered in portions during the day if desired.

The following examples give the preparation of certain dosage forms. Obviously any of the compounds of this invention can be used as the active ingredient indicated by the word "drug" in the formulations.

Capsules

| No. | Ingredient | mg/capsule | mg/capsule |
|---|---|---|---|
| 1. | Drug | 250 | 500 |
| 2. | Lactose USP | 100 | 50 |
| 3. | Corn Starch, Food Grade | 50 | 43.5 |
| 4. | Microcrystalline Cellulose | 95 | 50 |
| 5. | Magnesium Stearate NF | 5 | 6.5 |
| | Total | 500 | 650 |

Method of Manufacture

Mix Items Nos. 1, 2, 3 and 4 in a suitable mixer for 10-15 minutes. Add Item No. 5 and mix for 1-3 minutes. Fill the mixture into suitable two-piece hard gelatin capsules using suitable encapsulating machine.

Tablets

| No. | Ingredient | mg/capsule | mg/capsule |
|---|---|---|---|
| 1. | Drug | 250 | 500 |
| 2. | Lactose USP | 57 | 114 |
| 3. | Corn Starch, Food Grade, as a 10% paste in Purified Water | 20 | 40 |
| 4. | Corn Starch, Food Grade | 18 | 39 |
| 5. | Magnesium Stearate NF | 5 | 7 |
| | Total | 350 | 700 |

Method of Manufacture

Mix Items Nos. 1 and 2 in a suitable mixer for 10-15 minutes. Granulate the mixture with Item No. 3. Pass the wet granulation through a coarse screen (e.g., 1/4") if needed, and dry the wet granules. Mill the dried granules.

Combine Item No. 4 and the dried granules and mix for 10-15 minutes. Add Item No. 5 and mix for 1-3 minutes. Compress the mixture to appropriate size and weight on a suitable tablet machine.

Parenteral Dosage Forms

(a) Injection (Per vial)

| | mg/vial |
|---|---|
| Drug Sterile Powder | 500 |

Add sterile water for injection or bacteriostatic water for injection for reconstitution.

(b) Injectable Solution of drug

| Ingredient | mg/ml |
|---|---|
| Drug | 20 |
| Methylparaben | 0.2 |
| Propylparaben | 1.6 |
| Sodium Bisulfite | 3.2 |
| Disodium Edetate | 0.1 |
| Water for Injection q.s. ad | 1.0 ml |

Method of Manufacture

1. Dissolve parabens in a portion (85% of the final volume) of the water for injection at 65-70°C.
2. Cool to 25-35°C. Charge and dissolve the sodium bisulfite and disodium edetate.
3. Charge and dissolve Drug.
4. Bring the solution to final volume by adding water for injection.
5. Filter the solution through 0.22 membrane and fill into appropriate containers.
6. Terminally sterilize the units by autoclaving.

## C L A I M S

1. Compounds of the general formula I

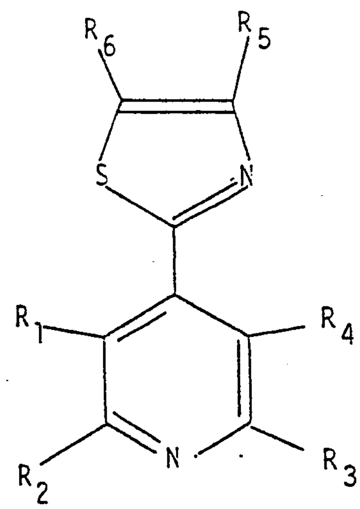

(I),

the pyridyl-N-oxides thereof and pharmaceutically acceptable salts of such compounds,

wherein $R_1$,$R_2$,$R_3$ and $R_4$ are independently selected from hydrogen, halogen, loweralkyl, loweralkoxy, hydroxy and amino; and

$R_5$ and $R_6$ are independently selected from hydrogen, loweralkyl, loweralkoxy, hydroxyloweralkyl, hydroxy, $-CF_3$, -CN, $-NR_7R_8$, 2-imidazolyl, $-\overset{Y}{\overset{\|}{C}}-NHOH$ and $-\overset{Y}{\overset{\|}{C}}-NR_{11}R_{12}$, with the provisos that

(i) $R_5$ and $R_6$ are not hydrogen simultaneously;

(ii) when $R_1,R_2,R_3,R_4$ and $R_6$ are all hydrogen, $R_5$ is not loweralkyl; and

(iii) when $R_1,R_2,R_3$ and $R_4$ are all hydrogen and $R_5$ is hydroxy, $R_6$ is not loweralkyl;

whereby, in the above definitions, $R_7$ and $R_8$ are independently selected from hydrogen and loweralkyl; $R_{11}$ and $R_{12}$ are independently selected from hydrogen, loweralkyl and hydroxyloweralkyl and Y represents O, S or NH.

2. Compounds according to claim 1 of the general formula II

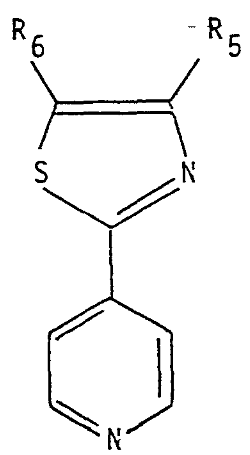

(II),

wherein one of $R_5$ and $R_6$ is hydrogen and the other is selected from -CN, 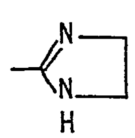 , 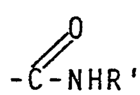 and 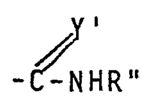

wherein R' is hydrogen, loweralkyl having from 2 to 6 carbon atoms or hydroxyloweralkyl having from 1 to 6 carbon atoms,

R" is hydrogen, hydroxy, loweralkyl having 1 to 6 carbon atoms or hydroxyloweralkyl having from 1 to 6 carbon atoms and

Y' is S or NH,

and pharmaceutically acceptable salts thereof.

3. Compounds according to claim 1, wherein one of $R_5$ and $R_6$ is hydrogen and the other is selected from -OH and $-NR_7R_8$ wherein $R_7$ and $R_8$ are as defined in claim 1.

4. Compounds according to claim 1 or 2, wherein one of $R_5$ and $R_6$ is hydrogen and the other is selected from 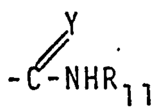, -CN, 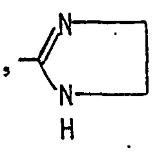, and 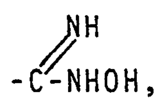, wherein $R_{11}$ and Y are as defined in claim 1.

5. Compounds according to claim 4 being

2-(4-pyridyl)-thiazole-4-thiocarboxamide;
2-(4-pyridyl)-thiazole-5-carboxamide;
2-(4-pyridyl)-thiazole-4-carboxamide;
N-(2-hydroxyethyl)-2-(4-pyridyl)-thiazole-4-carboxamide;
4-cyano-2-(4-pyridyl)-thiazole;
2-(4-pyridyl)-thiazole-4-carboxamidoxime;
2-(4-pyridyl)-thiazole-4-carboxamidine;
2-(4-pyridyl)-4-(2-imidazolin-2-yl)-thiazole;

and the pharmaceutically acceptable salts thereof.

6. A pharmaceutical composition comprising a compound as defined in any one of claims 1 to 5, in combination with a pharmaceutically acceptable carrier.

7. Process for the preparation of compounds of the general formula I as defined in claim 1, characterized in that

A: a compound of formula III

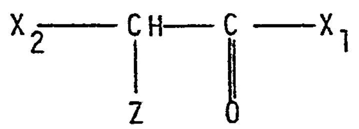

(III)

wherein Z is halogen and either one of $X_1$ and $X_2$ represents the group $-COOR_{14}$, wherein $R_{14}$ is hydrogen or loweralkyl, and the other represents $R_5$ respectively $R_6$, or both $X_1$ and $X_2$ represent the group $-COOR_{14}$, is reacted with a thioisonicotineamide of the formula IV

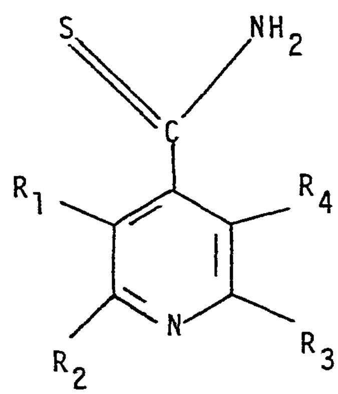

(IV)

wherein $R_1$,$R_2$,$R_3$ and $R_4$ are as defined in claim 1, yielding a compound of formula V

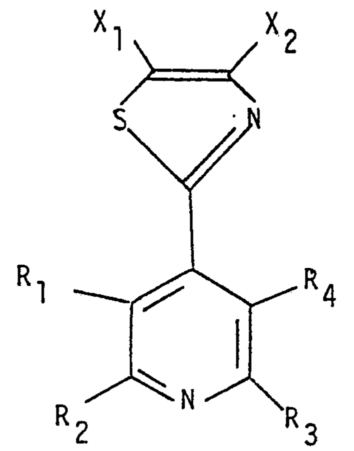

(V),

wherein $R_1, R_2, R_3, R_4, X_1$ and $X_2$ are as defined above; that any $-COOR_{14}$ group present in a compound of formula V, depending on the desired final compound, is transformed into one of the groups

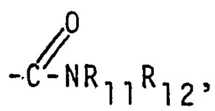, 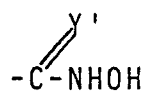 or $-NR_7R_8$

wherein $R_7, R_8, R_{11}$ and $R_{12}$ are as above defined and Y' is O or S; or

B: a compound of the general formula VI

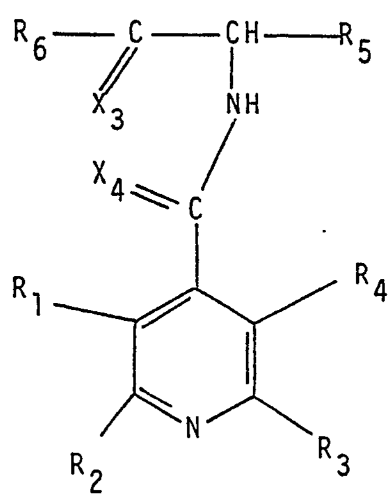

(VI)

wherein $R_1, R_2, R_3, R_4, R_5$ and $R_6$ are as defined in claim 1 and one of $X_3$ and $X_4$ represents S and the other O, is subjected to an intramolecular condensation;

and that a compound of formula I obtained according to process A or B above, if desired, is subjected to one or more of the following subsequent reactions at the substituents $R_5$ and/or $R_6$:

(i) alkylation of hydroxy to alkoxy;

(ii) transformation of $-\overset{O}{\overset{\|}{C}}-NR_{11}R_{12}$ into $-\overset{S}{\overset{\|}{C}}-NR_{11}R_{12}$;

(iii) transformation of $-\overset{O}{\overset{\|}{C}}-NH_2$ into CN;

(iv) transformation of CN into $-\overset{NH}{\overset{\|}{C}}-NR_{11}R_{12}$;

(v) transformation of CN into $-\overset{S}{\overset{\|}{C}}-NH_2$

(vi) transformation of CN into $-\overset{NH}{\overset{\|}{C}}-NHOH$ respectively $-\overset{NOH}{\overset{\|}{C}}-NR_{11}R_{12}$ ;

(vii) transformation of CN into 2-imidazolyl;

(viii) transformation of any compound of formula I into its 4-pyridyl-N-oxide;

(ix) transformation of a compound of formula I into its acid addition salt.

8. Process according to claim 7A, characterized in that the transformation of the ester group $-COOR_{14}$ wherein $R_{14}$ is as defined in claim 7, into the carboxamido group $-\overset{O}{\overset{\|}{C}}-NR_{11}R_{12}$ is accomplished by reacting a compound of formula V as defined in claim 7, with a compound of the general formula $HNR_{11}R_{12}$ wherein $R_{11}$ and $R_{12}$ are as defined in claim 1.

9. Process according to claim 7A, characterized in that the transformation of the ester group $-COOR_{14}$, wherein $R_{14}$ is as defined in claim 7, into a group $-\overset{Y'}{\overset{\|}{C}}-NHOH$ wherein Y' is as defined in claim 7 is accomplished by reacting a compound of formula V as defined in claim 7 with hydroxylamine or a salt thereof.

10. Process according to claim 7A, characterized in that the transformation of the ester group $-COOR_{14}$, wherein $R_{14}$ is as defined in claim 7, into a group of the formula $-NR_7R_8$ is accomplished by subjecting a compound of formula V as defined in claim 7 to a Curtis rearrangement comprising the following reaction steps:

(a) transformation of the $-COOR_{14}$ group into an acid hydrazide group $-\overset{O}{\overset{\|}{C}}-NHNH_2$ by treatment with $N_2H_4$; and

(b) transformation of the acyl hydrazide group into a group 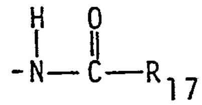 by treatment with nitrous acid or an alkali metal salt thereof in the presence of an acid anhydride, whereby $R_{17}$ represents hydrogen or loweralkyl;
followed, if desired, by

(c) alkylation of the group 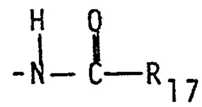 to a group 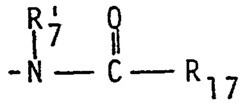 wherein $R_7'$ represents loweralkyl; and that

a compound obtained either according to step (b) or step (c) above is subjected to a reduction of the 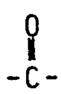 function to $-CH_2-$ or to an hydrolization whereby the 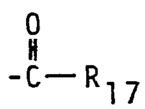 function is replaced by hydrogen.

11. Process for preparing pharmaceutical compositions, characterized in that a compound as defined in any one of claims 1 to 5 is brought into a form suitable for pharmaceutical administration.

# CLAIMS

1. Process for the preparation of compounds of the general formula I

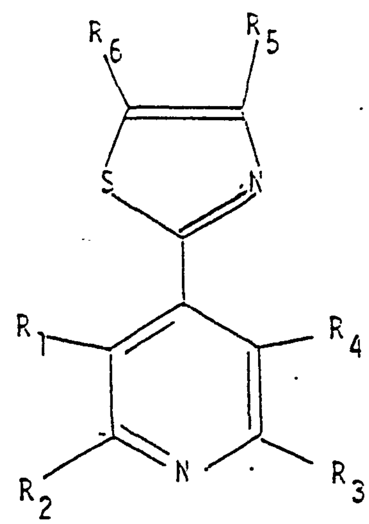

(I),

the pyridyl-N-oxides thereof and pharmaceutically acceptable salts of such compounds,

wherein $R_1$,$R_2$,$R_3$ and $R_4$ are independently selected from hydrogen, halogen, loweralkyl, loweralkoxy, hydroxy and amino; and

$R_5$ and $R_6$ are independently selected from hydrogen, loweralkyl, loweralkoxy, hydroxyloweralkyl, hydroxy, $-CF_3$, -CN, $-NR_7R_8$, 2-imidazolyl,

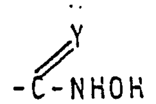 and 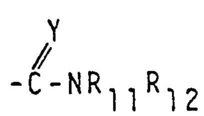, with the provisos that

(i) $R_5$ and $R_6$ are not hydrogen simultaneously;

(ii) when $R_1, R_2, R_3, R_4$ and $R_6$ are all hydrogen, $R_5$ is not loweralkyl; and

(iii) when $R_1, R_2, R_3$ and $R_4$ are all hydrogen and $R_5$ is hydroxy, $R_6$ is not loweralkyl;

whereby, in the above definitions, $R_7$ and $R_8$ are independently selected from hydrogen and loweralkyl; $R_{11}$ and $R_{12}$ are independently selected from hydrogen, loweralkyl and hydroxyloweralkyl and Y represents O, S or NH, characterized in that

A: a compound of formula III

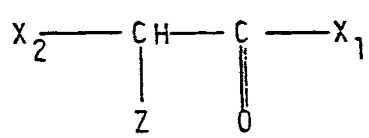

(III)

wherein Z is halogen and either one of $X_1$ and $X_2$ represents the group $-COOR_{14}$, wherein $R_{14}$ is hydrogen or loweralkyl, and the other represents $R_5$ respectively $R_6$, or both $X_1$ and $X_2$ represent the group $-COOR_{14}$, is reacted with a thioisonicotine-amide of the formula IV

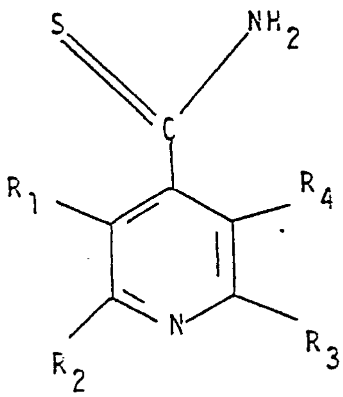

(IV)

wherein $R_1$,$R_2$,$R_3$ and $R_4$ are as defined above, yielding a compound of formula V

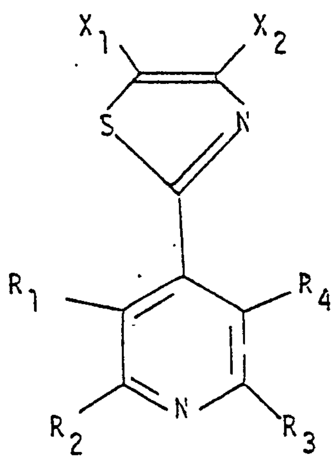

(V),

wherein $R_1$,$R_2$,$R_3$,$R_4$,$X_1$ and $X_2$ are as defined above; that any $-COOR_{14}$ group present in a compound of formula V, depending on the desired final compound, is transformed into one of the groups

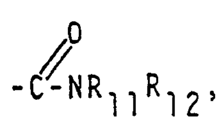, 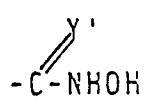 or $-NR_7R_8$

wherein $R_7$,$R_8$,$R_{11}$ and $R_{12}$ are as above defined and Y' is O or S; or

B: a compound of the general formula VI

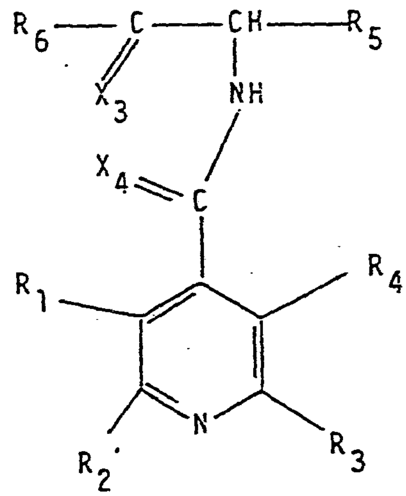

( VI )

wherein $R_1$,$R_2$,$R_3$,$R_4$,$R_5$ and $R_6$ are as defined in claim 1 and one of $X_3$ and $X_4$ represents S and the other O, is subjected to an intramolecular condensation; and

that a compound of formula I obtained according to process A or B above, if desired, is subjected to one or more of the following subsequent reactions at the substituents $R_5$ and/or $R_6$:

(i) alkylation of hydroxy to alkoxy;

(ii) transformation of 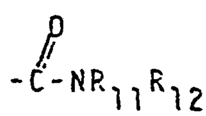 into 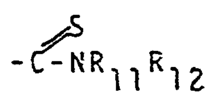;

(iii) transformation of 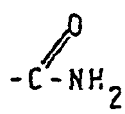 into CN;

(iv) transformation of CN into $-\overset{NH}{\overset{\|}{C}}-NR_{11}R_{12}$;

(v) transformation of CN into $-\overset{S}{\overset{\|}{C}}-NH_2$

(vi) transformation of CN into $-\overset{NH}{\overset{\|}{C}}-NHOH$ respectively $-\overset{NOH}{\overset{\|}{C}}-NR_{11}R_{12}$ ;

(vii) transformation of CN into 2-imidazolyl;

(viii) transformation of any compound of formula I into its 4-pyridyl-N-oxide;

(ix) transformation of a compound of formula I into its acid addition salt.

2. Process according to claim 1A, characterized in that the transformation of the ester group $-COOR_{14}$ wherein $R_{14}$ is as defined in claim 1, into the carboxamido group $-\overset{O}{\overset{\|}{C}}-NR_{11}R_{12}$ is accomplished by reacting a compound of formula V as defined in claim 1, with a compound of the general formula $HNR_{11}R_{12}$ wherein $R_{11}$ and $R_{12}$ are as defined in claim 1.

3. Process according to claim 1A, characterized in that the transformation of the ester group $-COOR_{14}$, wherein $R_{14}$ is as defined in claim 1, into a group $-\overset{Y'}{\overset{\|}{C}}-NHOH$ wherein Y' is as defined in claim 1 is accomplished by reacting a compound of formula V as defined in claim 1 with hydroxylamine or a salt thereof.

4. Process according to claim 1A, characterized in that the transformation of the ester group $-COOR_{14}$, wherein $R_{14}$ is as defined in claim 1, into a group of the formula $-NR_7R_8$ is accomplished by subjecting a compound of formula V as defined in claim 1 to a Curtis rearrangement comprising the following reaction steps:

(a) transformation of the $-COOR_{14}$ group into an acid hydrazide group $-\overset{O}{\overset{\|}{C}}-NHNH_2$ by treatment with $N_2H_4$; and

(b) transformation of the acyl hydrazide group into a group 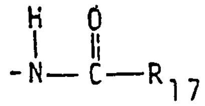 by treatment with nitrous acid or an alkali metal salt thereof in the presence of an acid anhydride, whereby $R_{17}$ represents hydrogen or loweralkyl;
followed, if desired, by

(c) alkylation of the group 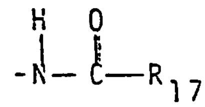 to a group 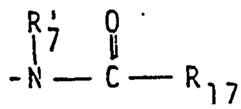 wherein $R_7'$ represents loweralkyl;

and that
a compound obtained either according to step (b) or step (c) above is subjected to a reduction of the 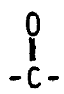 function to $-CH_2-$ or to an hydrolization whereby the 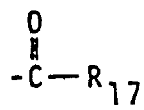 function is replaced by hydrogen.

5. Process according to claim 1, characterized in that compounds of formula II

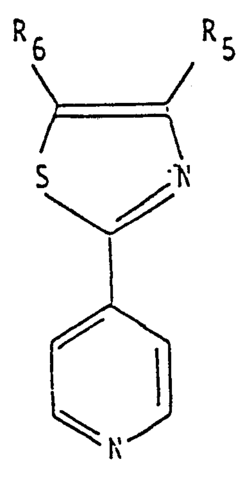

(II)

wherein one of $R_5$ and $R_6$ is hydrogen and the other is

selected from -CN, 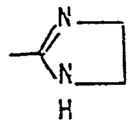, 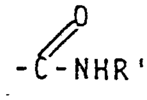 and 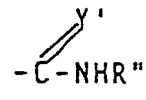

wherein R' is hydrogen, loweralkyl having from 2 to 6 carbon atoms or hydroxyloweralkyl having from 1 to 6 carbon atoms,

R" is hydrogen, hydroxy, loweralkyl having 1 to 6 carbon atoms or hydroxyloweralkyl having from 1 to 6 carbon atoms and

Y' is S or NH,

and pharmaceutically acceptable salts thereof, are prepared.

6. Process according to claim 1, characterized in that compounds of formula I wherein one of $R_5$ and $R_6$ is hydrogen and the other is selected from -OH and $-NR_7R_8$ wherein $R_7$ and $R_8$ are as defined in claim 1, are prepared.

7. Process according to claim 1, characterized in that compounds wherein one of $R_5$ and $R_6$ is hydrogen and the other is selected from 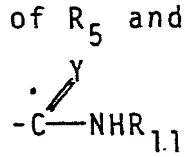, -CN, 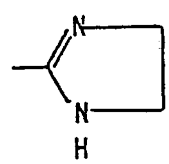 and 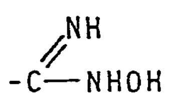, wherein $R_{11}$ and Y are as defined in claim 1, are prepared.

8. Process according to claim 5 characterized in that the compounds

2-(4-pyridyl)-thiazole-4-thiocarboxamide;
2-(4-pyridyl)-thiazole-5-carboxamide;
2-(4-pyridyl)-thiazole-4-carboxamide;
N-(2-hydroxyethyl)-2-(4-pyridyl)-thiazole-4-carboxamide;
4-cyano-2-(4-pyridyl)-thiazole;
2-(4-pyridyl)-thiazole-4-carboxamidoxime;
2-(4-pyridyl)-thiazole-4-carboxamidine;
2-(4-pyridyl)-4-(2-imidazolin-2-yl)-thiazole;

and the pharmaceutically acceptable salts thereof, are prepared.

9. Process for preparing pharmaceutical compositions, characterized in that a compound as defined in any one of claims 1 to 7, are brought into a form suitable for pharmaceutical administration.